# EUROPÄISCHE PATENTSCHRIFT

(11) **EP 3 458 571 B1**
(45) Veröffentlichungstag und Bekanntmachung des Hinweises auf die Patenterteilung: **05.07.2023**
(21) Anmeldenummer: 17733960.3
(22) Anmeldetag: 12.05.2017
(51) Int. Cl.: C12N 5/071, G01N 33/50

(54) **VERFAHREN ZUR BILDUNG VON NIERENTUBULI**
PROCESS FOR GENERATING RENAL TUBULES
PROCEDE POUR GENERER TUBULES DE REIN

(30) Priorität: 17.05.2016 DE 102016109067
(43) Veröffentlichungstag der Anmeldung: 27.03.2019
(73) Patentinhaber: Leibniz-Institut für Polymerforschung Dresden e.V., 01069 Dresden (DE)
(72) Erfinder: WEBER, Heather, Boston, MA 02215 (US); TSURKAN, Mikhail, 01217 Dresden (DE); MAGNO,Dr. Valentina, 01099 Dresden (DE); FREUDENBERG, Uwe, 01217 Dresden (DE); WERNER, Carsten, 01324 Dresden (DE)
(74) Vertreter: Sperling, Thomas
(86) Internationale Anmeldenummer: PCT/DE2017/100407
(87) Internationale Veröffentlichungsnummer: WO 2017/198257

(56) Entgegenhaltungen:
- EP-A1- 2 078 073
- WO-A1-2015/069192
- Masaaki Miya ET AL: "Enhancement of in vitro human tubulogenesis by endothelial cell-derived factors: implications for in vivo tubular regeneration after injury", American Journal of Physiology - Renal Physiology, 1. August 2011 (2011-08-01), Seiten 387-395, XP055393899, United States DOI: 10.1152/ajprenal.00619.2010 Gefunden im Internet: URL:http://ajprenal.physiology.org/content /ajprenal/301/2/F387.full.pdf [gefunden am 2017-07-26]
- ANNA I ASTASHKINA ET AL: "Comparing predictive drug nephrotoxicity biomarkers in kidney 3-D primary organoid culture and immortalized cell lines", BIOMATERIALS, ELSEVIER SCIENCE PUBLISHERS BV., BARKING, GB, Bd. 33, Nr. 18, 3. März 2012 (2012-03-03), Seiten 4712-4721, XP028411046, ISSN: 0142-9612, DOI: 10.1016/J.BIOMATERIALS.2012.03.001 [gefunden am 2012-03-15] in der Anmeldung erwähnt
- BALAJIKARTHICK SUBRAMANIAN ET AL: "Tissue-Engineered Three-Dimensional In Vitro Models for Normal and Diseased Kidney", TISSUE ENGINEERING PART A, Bd. 16, Nr. 9, 1. September 2010 (2010-09-01), Seiten 2821-2831, XP055393901, US ISSN: 1937-3341, DOI: 10.1089/ten.tea.2009.0595
- MIKHAIL V. TSURKAN ET AL: "Defined Polymer-Peptide Conjugates to Form Cell-Instructive starPEG-Heparin Matrices In Situ", ADVANCED MATERIALS, Bd. 25, Nr. 18, 14. Mai 2013 (2013-05-14), Seiten 2606-2610, XP055393137, DE ISSN: 0935-9648, DOI: 10.1002/adma.201300691 in der Anmeldung erwähnt
- TSURKAN MIKHAIL V ET AL: "Growth factor delivery from hydrogel particle aggregates to promote tubular regeneration after acute kidney injury", JOURNAL OF CONTROLLED RELEASE, ELSEVIER, AMSTERDAM, NL, Bd. 167, Nr. 3, 8. Februar 2013 (2013-02-08), Seiten 248-255, XP028581018, ISSN: 0168-3659, DOI: 10.1016/J.JCONREL.2013.01.030
- TERESA M. DESROCHERS ET AL: "Bioengineered 3D Human Kidney Tissue, a Platform for the Determination of Nephrotoxicity", PLOS ONE, Bd. 8, Nr. 3, 14. März 2013 (2013-03-14), Seite e59219, XP055393974, DOI: 10.1371/journal.pone.0059219 in der Anmeldung erwähnt
- DESROCHERS TERESA M ET AL: "Tissue-engineered kidney disease models", ADVANCED DRUG DELIVERY REVIEWS, Bd. 69, 17. Dezember 2013 (2013-12-17), Seiten 67-80, XP028648695, ISSN: 0169-409X, DOI: 10.1016/J.ADDR.2013.12.002
- WEBER HEATHER M ET AL: "Heparin-based hydrogels induce human renal tubulogenesisin vitro", ACTA BIOMATERIALIA, ELSEVIER, AMSTERDAM, NL, Bd. 57, 17. Mai 2017 (2017-05-17), Seiten 59-69, XP085092736, ISSN: 1742-7061, DOI: 10.1016/J.ACTBIO.2017.05.035

## Beschreibung

Die Erfindung betrifft ein Verfahren zur Bildung von Nieren-Tubuli und damit die Etablierung eines ex-vivo Modells für Nierentubuli (Nierenkanälchen), mit dem Entwicklungsprozesse studiert und Arzneimitteltoxizitätstests durchgeführt werden können.

Die Nephrone sind eine funktionelle Untereinheit der Niere, welche aus Nierenkörperchen und -Tubuli bestehen. Die Nierentubuli, insbesondere die proximalen Tubuli, spielen eine wichtige Rolle bei der Ausscheidung von Stoffwechselprodukten (Giftstoffen) und der Reabsorption von Nährstoffen aus dem Harn. Die proximalen Tubuli reagieren entsprechend sehr sensitiv auf über die Niere ausgeschiedene Stoffwechselprodukte und Arzneimittel.

Gegenwärtig angewandte Nephrotoxizitätsassays basieren entweder auf zweidimensionalen in vitro Kulturen oder Tiermodellen. Die 2D-Kultur von Zellen ist bedingt durch den fehlenden Zell-Zell und Zell-Matrix-Kontakt aber sehr artifiziell und ist daher von dem physiologischen relevanten Umfeld weit entfernt. Aus diesem Grund werden Nierentoxizitätsstudien zumeist im Tiermodell durchgeführt. Diese Studien zeigen jedoch in ca. 50% der Fälle gegensätzliche Trends im Vergleich zur menschlichen Reaktion für Arzneimittel, wie zum Beispiel aus der Druckschrift Archibald K, Coleman R, Foster C. Open letter to UK Prime Minister David Cameron and Health Secretary Andrew Lansley on safety of medicines. Lancet. 2011;377:1915**,** hervorgeht.

Aus diesem Grund wurden nierenspezifische Organoid-Assays als Ersatz für die weit verbreiteten Nierentoxizitätsassays entwickelt, wie bei Davies J. Engineered renal tissue as a potential platform for pharmacokinetic and nephrotoxicity testing. Drug Discovery Today (2014**),** beschrieben. Dabei konnte gezeigt werden, dass 3D-Organoidmodelle im Vergleich zur konventionellen Verwendung imortalisierter Zellinien (2D Kultur) besser die nephrotoxischen Effekte in vivo nachvollziehen lassen **(**Astashkina A.I. et al. Comparing predictive drug nephrotoxicity biomarkers in kidney 3-D primary organoid culture and immortalized cell lines. Biomaterials (2012) 33 4712-21**).**

Darüber hinaus ist, unter anderem aus der Druckschrift Astashkina A.I. et al. A 3-D organoid kidney culture model engineered for high-throughput nephrotoxicity assays. Biomaterials (2012**),** ein zumindest teilweise auf synthetischen Materialen basierendes renales Tubulimodell bekannt, welches Hyaluronsäure (HA) und Polyethylenglycoldiacrylate (PEGDA) nutzt sowie ein Tubulimodell basierend auf einem porösem Hydrogelen aus Seidenproteinen gefüllt mit einer Mischung aus Kollagen und Matrigel^{™}. Das auf porösen Hydrogelen basierende Modell nutzte gesunde oder erkrankungstypisch veränderte embryonale murine Nierenepithelzellen. Das Modell basierend auf HA-PEGDA zeigte vielversprechende Ergebnisse mit High-Throughputkompatiblen Assays. Insgesamt haben die 3D-Studien Anhaltspunkte für die Relevanz von 3D-Zellkulturmodellen im Nierenkontext erbracht.

Weiterhin wurden renale 3D-Tubulimodelle auf Basis von Kollagen, Matrigel^{™} oder Kombinationen aus beiden Biopolymerpräparationen entwickelt, wie unter anderem bei DesRochers, T.M., Suter, L., Roth, A., Kaplan, D. Bioengineered 3D Human Kidney Tissue, a Platform for the Determination of Nephrotoxicity. Plos One 8, (2013**),** beschrieben.

Aus Masaaki Miya ET AL : "Enhancement of in vitro human tubulogenesis by endothelial cell-derived factors : implications for in vivo tubular regeneration after injury", American Journal of Physiology - Renal Physiology, 1. August 2011 (2011-08-01), S. 387-395 XP055393899, United States DOI: 10.1152/ajprenal.00619.2010**,** ist die Kultur von humanen tubulären Epithelzellen der Niere in dreidimensionalen Gelen aus Matrigel^{™} und Collagen I bekannt, wobei die tubuläre Struktur durch einen Wachstumsfaktor induziert wird.

Nachteilig am Stand der Technik sind neben den oben genannten Einschränkungen in Bezug auf die artifizielle 2D-Umgebung in konventioneller Zellkultur oder die teilweise wenig aussagekräftigen bzw. aufgrund großer biologischer Unterschiede sogar gegenläufig reagierenden Tiermodelle bei den bisher beschriebenen Organoidmodellen folgende:
Die Organoidmodelle verwenden Zellen tierischen Ursprungs sowie embryonale oder induziert pluripotente Stammzellen, welche aufgrund von Speziesunterschieden, aus ethischen Gründen, und wegen der Gefahr der Entstehung eines Teratoms von nur eingeschränktem Nutzen sind. Darüber hinaus weisen die genutzten biopolymerbasierten Matrixsysteme starke chargenabhängige Schwankungen auf und sind daher schlecht reproduzierbar anwendbar. Außerdem ist keine definierte Variation der mechanischen und biomolekularen Eigenschaften und somit ist auch keine Untersuchung dieser Parameter auf die Tubulogenese möglich.

Biologisch von Nachteil ist zudem, dass die Dimensionen, Form und Morphologie der tubuliartigen Strukturen deutlich abweichend von dem der angestrebten humanen Nierentubuli sind.

Das HA-PEGDA-Hydrogel im Speziellen nutzt zudem Fragmente von proximalen Tubuli, gewonnen aus Mäusenieren, und ist daher nicht geeignet die humane Situation zu beschreiben beziehungsweise den Prozess der humanen Tubulogenese zu studieren.

Die Aufgabe der Erfindung bestand darin, ein definiertes und in den Eigenschaften modulierbares dreidimensionales Modellsystem humaner Tubuli zu erzeugen, welche in der Struktur und Funktion dem Vorbild der humanen proximalen Nierentubuli sehr nahe kommt und die Bildung der Tubuli und/oder deren Reaktion auf Arzneimittel untersuchen lässt.

Das Modellsystem sollte robust und langzeitstabil sein, um reproduzierbare Studien über längere Zeiträume, das heißt zumindest über 4-6 Wochen, zu ermöglichen. Außerdem sollte der Einfluss der unterschiedlichen zellinstruktiven Signale, wie mechanische Eigenschaften, Umbaubarkeit, Präsentation löslicher Signalmoleküle, der natürlichen Zellumgebung, der sogenanten extrazellulären Matrix beziehungsweise auf die Tubulogenese und die Arzneimittelwirkung auch durch Nutzung aussagekräftiger Assays, zum Beispiel hinsichtlich metabolischer Aktivität und Toxizität sowie renaler Erkankungsmarker, sowie erweiterter Einzelzellanalysen, zum Beispiel zur Morphologie, Immunochemistry, Durchflusszytometrie, PCR, Western Blot, charakterisierbar sein. Die Reaktion auf die Gabe von Wirkstoffen und Medikamenten sollte dabei weitgehend vergleichbar mit den von etablierten in-vivo-Tests sein und somit helfen, kostspielige und ethisch bedenkliche Tierversuche einzuschränken.

Außerdem sollen Arraytechniken eine Aufskalierung von Breitbandstests (Screening) ermöglichen.

Die Aufgabe der Erfindung wird mit einem Verfahren mit den Merkmalen des Anspruchs 1 gelöst. Weiterbildungen sind in den Unteransprüchen angegeben.

Im erfindungsgemäßen Verfahren zur Bildung von Nieren-Tubuli erfolgt eine Einbettung von einzelnen Nieren-Zellen in ein synthetisches Hydrogel, basierend auf Polyethylenglykol (PEG) als Komponente, und die Kultivierung der Zellen bis zur Bildung von Tubulistrukturen.

Gemäß der Erfindung ist/wird das PEG-Hydrogelsystem über enzymatisch spaltbare Peptide, vorzugsweise durch Matrix-Metalloproteinasen spaltbare Peptide (MMP-Peptide), vernetzt, wodurch das Hydrogelsystem spaltbar und lokal umbaubar ist.

Dabei können die Polyethylenglykolmoleküle miteinander durch die konjugierten Peptide zu einem PEG-Peptid/PEG-System vernetzt werden und somit PEG-Peptid/PEG-Hydrogele bilden. Bevorzugt sind dabei PEG-MMP/PEG-Hydrogele. Vorzugsweise wird als Polyethylenglykol ein mehrarmiges Polyethylenglykol eingesetzt. Bevorzugt wird dabei das vierarmige Polyethylenglykol (Stern-PEG).

Gemäß der Erfindung werden die PEG-Moleküle mit Molekülen eines Glykosaminoglykans, ausgewählt aus Heparin, aus Heparin mit angepasstem Sulfatierungsmuster, Chondroitinsulfat und Hyaluronsäure, durch die Peptidmoleküle miteinander vernetzt und bilden Hydrogele. Bevorzugt werden bei dieser Ausführungsvariante PEG-MMP/Heparin-Hydrogelsysteme verwendet, als besonders vorteilhaft erwiesen sich Stern-PEG-MMP/Heparin-Hydrogelsysteme. In einer Ausführungsvariante wird die Hydrogelmatrix des Hydrogels durch eine kovalente Vernetzung eines thiolterminierten Stern-PEG-Peptid-Konjugats und eines durch Maleimid, vorzugsweise durch 4-6 Maleimidgruppen, funktionalisierten Heparins gebildet. Dabei erfolgt die Vernetzung der Hydrogelmatrix über eine Michael-Addition.

Die für die Kultur verwendeten Nieren-Zellen entstammen vorzugsweise Nierentubuluszelllinien oder sind primäre Nierentubuluszellen oder aus induziert pluripotenten Stammzellen (iPSCs) oder von mesenchymalen Stammzellen abgeleitete Zellen.

Besonders vorteilhaft ist es dabei, wenn die Nieren-Zellen humanen Nierentubuluszelllinien entstammen oder humane primäre Nierentubuluszellen sind oder aus humanen induziert pluripotenten Stammzellen (iPSCs) oder von humanen mesenchymalen Stammzellen abgeleitete Zellen sind.

Die Nierentubuluszellen können zum Beispiel proximale Tubuluszellen, distale Tubuluszellen oder Sammelrohrzellen sein. Die primären Nierentubuluszellen sind vorzugsweise humane primäre proximale Nierentubuluszellen.

Die Nierentubulus-Zelllinie ist vorteilhafterweise eine immortalisierte proximale Tubulus-Epithelzelllinie, abgeleitet von normalem adultem humanen Nierengewebe.

Die Kultivierung wird vorzugsweise solange fortgesetzt, bis die Tubulistrukturen in Größe, Struktur und Morphologie und Funktionalität den adulten humanen Nierentubuli entsprechen oder zumindest ähneln.

Die Funktionalität der Nierntubuli ist zum Beispiel durch die Expression humaner renaler Marker, vorzugsweise Polarisationsmarker, die Ansprechbarkeit auf bekannte nephrotoxische Verbindungen, zum Beispiel Cis-Platin, und daraus folgender Expression renaler Verletzungsmarker, zum Beispiel des renalen Verletzungsmarkers KIM1, und Apoptose-Marker, zum Beispiel abgespaltete Caspase-3, und dadurch, dass die Tubuli in der Lage sind, anionisch geladene organische Moleküle in das Lumen der Tubuli zu transportieren, beschreibbar.

Entsprechend einer Ausgestaltung der Erfindung erfolgt eine Co-Kultivierung der Tubuluszellen zusammen mit mesenchymalen Stammzellen, Nierenzellen oder Endothelzellen, die co-lokalisiert mit den Tubuluszellen im Hydrogel vorliegen.

Besonders bevorzugt ist dabei eine Co-Kultivierung der Tubuluszellen zusammen mit humanen mesenchymalen Stammzellen und/oder humanen Nierenzellen und/oder humanen Endothelzellen, die co-lokalisiert mit den Tubuluszellen im Hydrogel vorliegen.

Das Verfahren kann mit und ohne Serum, das heißt 0 % (v/v) Serum, durchgeführt werden.

Durch das Verfahren sind humane Nierentubuli in einer dreidimensionalen Hydrogelmatrix erhältlich, die in Größe, Struktur und Morphologie und Funktionaliät adulten humanen Nierentubuli entsprechen oder zumindest ähneln.

Das Verfahren eignet sich zur Anwendung zum dreidimensionalen Monitoring der Bildung von humanen Nierentubuli oder Nierentubuli von Säugetieren, zum Beispiel von Mäusen oder Ratten.

Besonders vorteilhaft ist dabei die Anwendung zur Charakterisierung des Einflusses der Hydrogelsteifigkeit und/oder des Einflusses des Abbaus des Hydrogels über enzymatisch spaltbare Peptidbrücken, vorzugsweise MMP, und/oder die Charakterisierung des Einflusses der Gegenwart von Glykosaminoglykanen, wie zum Beispiel Heparin, und/oder Peptiden, vorzugsweise von Proteinen der extrazellulären Matrix (EZM) abgeleiteten Signalpeptide, auf die Bildung der Nierentubuli durch Einbau der Glykosaminoglykane und/oder Peptide in das synthetische Hydrogel.

Dabei kann das Verfahren zur Charakterisierung des Einflusses von löslichen Molekülen oder Wirkstoffen, wie Signalmolekülen, insbesondere Wachstumsfaktoren, auf die Bildung der Nierentubuli durch Zugabe einer Verbindung zum Kulturmedium oder Einbettung der Komponente in das Hydrogel angewendet werden.

Besonders bevorzugt ist die Anwendung des Verfahrens für die Analyse der Toxizität einer Verbindung auf Nierentubuli durch Zugabe dieser Verbindung zum Medium oder deren Einbetten in das Hydrogel.

Die Untersuchungen der Tubulogenese oder der Nephrotoxizität können vorteilhafterweise unter Durchführung des Verfahrens einer 24-, 48- oder 96-Wellplatte erfolgen.

Weitere Einzelheiten, Merkmale und Vorteile von Ausgestaltungen der Erfindung ergeben sich aus der nachfolgenden Beschreibung von Ausführungsbeispielen mit Bezugnahme auf die zugehörigen Zeichnungen. Es zeigen:
- **Fig. 1:**: den experimentellen Aufbau eines Ex-vivo-Nierentubuli-Assays,
- **Fig. 2A:**: ein Hydrogel mit primären proximalen Zellen an einem Metallspatel nach vier Wochen in der Kultur,
- **Fig. 2B:**: mikroskopische Aufnahmen von Nierenstrukturen nach 4 Wochen in der Kultur von HK-2-Zellen in verschiedenen Hydrogelsystemen,
- **Fig. 3A:**: mikroskopische Aufnahmen, die die Polarisation von HK-2-Zellen nach vier Wochen in der Zellkultur zeigen,
- **Fig. 3B:**: mikroskopische Aufnahmen, die die Immunofluoreszenz von Polarisationsmarkern auf primären humanen proximalen Tubuluszellen nach vier Wochen in der Zellkultur zeigen,
- **Fig. 4:**: mikroskopische Aufnahme mit Phasenkontrast und Fluoreszenzbildern der Nierentubuli im Hydrogel vor und nach der Inkubation mit einem anionischen, organischen Fluoreszenzfarbstoff,
- **Fig. 5:**: ein Diagramm, das bei Verwendung von HK-2-Zellen die metabolische Aktivität und Zytotoxizität nach 48 Stunden Inkubation mit Cis-Platin zeigt,
- **Fig. 6:**: mikroskopische Aufnahmen, die bei Verwendung von HK-2-Zellen die Expression eines Biomarkers für Nierenschädigung, KIM-1, nach Inkubation mit oder ohne Cis-Platin zeigen,
- **Fig. 7:**: ein Diagramm mit der Quantifizierung der Tubulogenese bei Verwendung von HK-2-Zellen mit 10% fötalem Rinderserum (FBS) und 10% Pannexine NTA Serumersatz (NTA) im Vergleich, und
- **Fig. 8:**: die metabolische Aktivität der Kultur von HK-2-Zellen mit fötalem Rinderserum (FBS) und der Kultur mit 10% Pannexine NTA Serumersatz (NTA) im Vergeich, gemessen mit Presto Blau-Assay in der dritten Woche der Zellkultur.

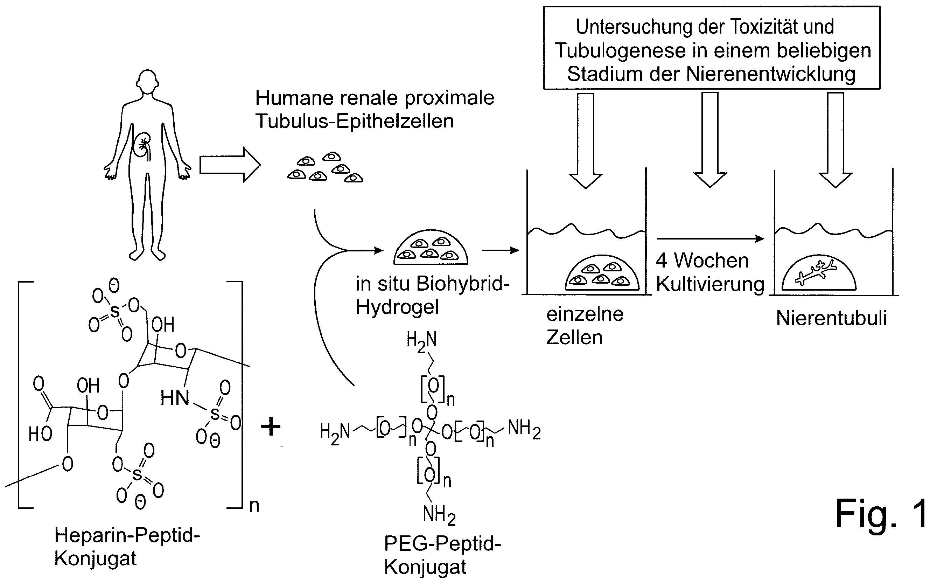

Das in **Fig. 1** gezeigte Verfahren basiert auf der Verwendung von Hydrogelen, die aus vernetztem vierarmigen Polyethylenglycol (Stern-PEG) und Heparin, welches mit mehreren Maleimidgruppen funktionalsiert ist, gebildet werden. Die Bildung von solchen Hydrogelen wurde beispielsweise bereits bei Tsurkan M.V. et al. Defined Polymer-Peptide Conjugates to Form Cell-Instructive starPEG-Heparin Matrices In Situ. Advanced Materials (2013**)** beschrieben. Stern-PEG wurde in der Weise modifiziert, dass es ein durch Matrix-Metalloproteasen (MMP) spaltbares Peptid einschließt, um einen zellkontrollierten lokalen Abbau- und Umbau der Hydrogelmatrix zu ermöglichen. Heparin-Maleimid und Stern-PEG werden beide in phosphatgepufferter Kochsalzlösung (PBS) gelöst. Durch Mischung der beiden Lösungen bildet sich in weniger als einer Minute ein Hydrogel durch einen Michael-Typ-Addition zwischen Heparin-Maleimid und den thiol-terminierten PEG-Peptid-Konjugaten oder dem thiolterminierten PEG. Humane proximale Tubulus-Epithelzellen wurden in das Hydrogel schon durch ein erstes Einmischen in die Heparin-Maleimid-Lösung mit Hilfe einer Pipette und einem anschließenden Mischen der Heparin-Maleimid-Zellmischung mit der Stern-PEG-Lösung eingebettet.

Humane proximale Niernentubili-Epithelzellen der Zelllinie HK-2 (ATCC CRL-2190) wurden in DMEM/F-12-Medium der Firma Gibco, ergänzt mit 10 % Fötalem Rinderserum (FBS) der Firma Biochrom) und 1 % Penicillin/Streptomycin-Lösung, kultiviert. Die HK-2-Zellen wurden in die Hydrogele, 50.000 Zellen pro Hydrogel, eingebettet und vier Wochen lang kultiviert. Das Medium wurde aller drei Tage gewechselt. Zur Tubuli-Bildung kam es etwa nach drei Wochen.

Die **Fig. 1** zeigt, schematisch, ein Heparin-Peptid-Konjugat und ein PEG-Peptid-Konjugat mit den humanen renalen Epithelzellen (HK-2), zu einem Biohybrid-Hydrogel zusammengemischt werden. Die Kultivierung der Zellen erfolgt bis zur Bildung von Tubulistrukturen. Nach vier Wochen in der Kultur haben sich bereits Nierentubuli gebildet. Über den gesamten Zeitraum der Kultur können Untersuchungen zur Toxizität und Tubulogenese in einem beliebigen Stadium der Tubuli-Entwicklung durchgeführt werden.

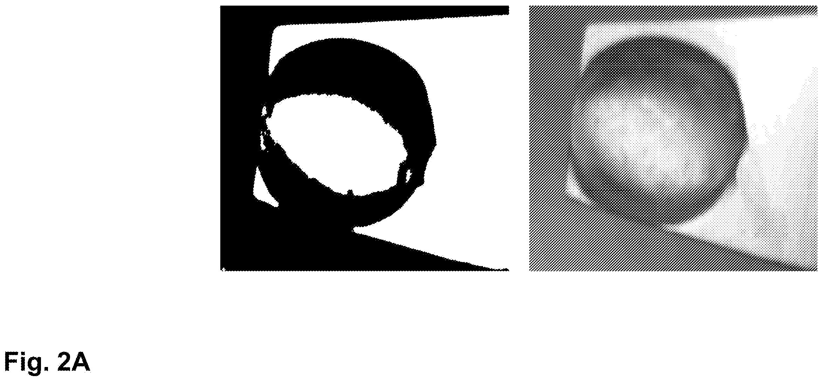

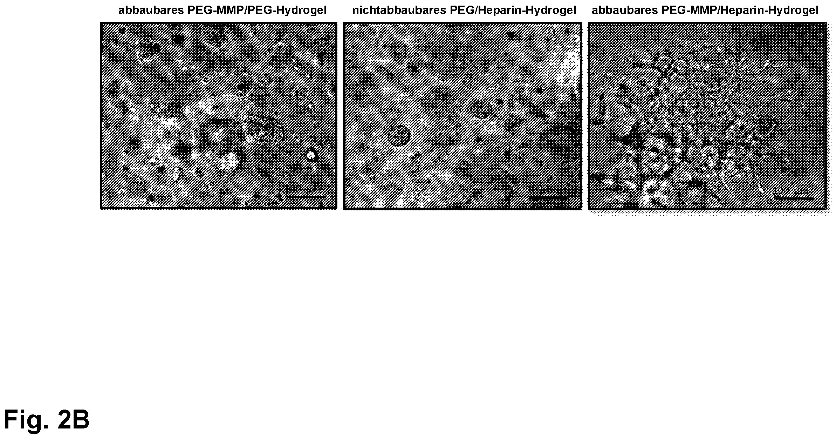

Die **Fig. 2** **A** zeigt ein Hydrogel mit primären proximalen Zellen an einem Metallspatel nach vier Wochen in der Kultur. Die Hydrogele, die stabile Scheiben bilden, sind dann leicht bearbeitbar.

Hydrogele können auch aus Heparin mit angepasstem Sulfatierungsmuster oder anderen Glykosaminoglykanen, ausgewählt aus Hyaluronsäure und Chondroitinsulfat, hergestellt werden. Die **Fig. 2B** zeigt mikroskopische Aufnahmen von drei verschiedenen Hydrogelsysteme mit HK-2-Zellen, ein abbaubares PEG-MMP/PEG-Hydrogel, ein nichtabbaubares (nicht-spaltbares) PEG/Heparin-Hydrogel sowie ein abbaubares PEG-MMP/Heparin-Hydrogel. Dabei wurden die Ergebnisse der Kultivierung gegenübergestellt. Die besten Ergebnisse bezüglich der Ausbildung von Nierentubuli konnten in dem abbaubaren PEG-MMP/Heparin-Hydrogel erzielt werden.

Die mechanischen Eigenschaften dieser Hydrogele können leicht durch Einstellen des Mischungsverhältnisses der beiden Materialkomponenten, also dem molaren Verhältnis von PEG-MMP zu Heparin, das heißt des Vernetzungsgrades, abgestimmt werden. Dies erzeugt ein modular einstellbares System, in dem die mechanischen Eigenschaften (Steifigkeit) des Systems bei der Tubulogenese getestet werden können. Variationen der Steifigkeit sind dabei, ausgedrückt durch das Speichermodul, von 200 Pa bis 6 kPa möglich. Das Speichermodul kann vorzugsweise mittels oszillatorischer Rheometrie bestimmt werden. Zusätzlich können Peptide der extrazelluläre Matrix (EZM) an das PEG oder Heparin gebunden werden, um ihre Wirkung auf Tubulogenese zu untersuchen. Die negative Ladung des Heparins kann genutzt werden, um lösliche Faktoren, wie zum Beispiel Wachstumsfaktoren oder Zytokine, elektrostatisch zu binden. Diese Faktoren können dann im Laufe der Zeit freigesetzt werden, um die Dynamik der In-vivo-Umgebung zu simulieren. Auf diese Weise könnten mehrere Faktoren gleichzeitig hinsichtlich ihrer Wirkung auf die Nieren-Tubulogenese erforscht werden.

Es wurden Untersuchungen mit humanen proximalen Tubulus-Zelllinien und humanen primären proximalen Tubuluszellen durchgeführt. Zellen in den abbaubaren PEG-MMP/Heparin-Hydrogelen bildeten röhrenförmige Strukturen, die anatomisch, physiologisch und funktionelle Eigenschaften aufweisen, die den Eigenschaften von in vivo humanen proximalen Tubuli ähnlich sind. In keinem der bisher bekannten In-vitro-Verfahren konnten humane proximale Tubuli in vitro mit einem Durchmesser erzeugt werden, der dem Durchmesser von in vivo humanen proximalen Tubuli ähnlich ist. Die Strukturen weisen auch die klassischen Polarisationsmarker und extrazellulären Matrixkomponenten auf, die auch in den in vivo proximalen Nierentubuli gefunden wurden.

Die Vorteile der Erfindung gegenüber dem oben genannten Stand der Technik sind die Folgenden:
- ein humanes Zellsystem,
- Tubulus-Strukturen liegen im anatomischen Größenbereich,
- Tubulus-Strukturen werden polarisiert wie in vivo,
- röhrenförmige Strukturen sind funktionell (transportieren anionische organische Moleküle, sprechen auf nephrotoxische Wirkstoffe) an,
- Langlebigkeit - sie können für mindestens 5 Wochen kultiviert werden,
- Einstellbarkeit des Materials, um die Auswirkungen der verschiedenen zellinstruktiven Signale zu untersuchen,
- ein optisch klares Material für Zellaufnahmen und Färbungen
- die Anwendbarkeit für personalisierte Medizin mit Nutzung von primären humanen Zellen eines Patienten,
- Wegfall der Notwendigkeit von Tierversuchen,
- Hydrogele können abgebaut und die Zellen für die quantitative Analyse (FACS, PCR, Western-Blot, IHC) oder Retransplantation verwendet werden,
- Verfahren kann als Hochdurchsatz -Assay verwendet werden,
- sowohl die metabolische Aktivität als auch die Zytotoxizität können leicht quantifiziert werden.

Der wesentliche Vorteil des Systems besteht darin, dass die aufgrund der synthetischen, am MMP-Peptid spaltbaren Stern-PEG-MMP/Heparin-Hydrogele ein Verfahren für die Ex-vivo-Herstellung eines Nierentubulus ermöglichen. Dieses Biohybrid-Hydrogel stellt eine stabiles, einstellbares System bereit, in dem die Tubulogenese direkt für Zwecke des Gewebe-Engineering gesteuert werden kann. Darüber hinaus, im Gegensatz zu einigen der oben genannten Verfahren aus dem Stand der Technik, kann dieses Verfahren menschliche Zellen verwenden, es ist somit als ein direktes Modell für menschliche Nierentubulogenese geeignet. Aus diesem Grund bietet es eine hervorragende Alternative zu Nephrotoxizitätsuntersuchungen an Tieren oder zu 2D-Zellkulturmodellen. Auch können mit diesem Verfahren Zellen von Patienten für personalisierte Toxizitätsstudien verwendet werden.

Im Gegensatz dazu werden in vielen der oben genannten, aus dem Stand der Technik bekannte Verfahren Nierentubuli-artige Strukturen aus tierischen Zellen oder Stammzellen oder von humanen Zellen abgeleitete Strukturen erzeugt, die um Größenordnungen kleiner als die humanen proximalen Nieren-Tubuli und oft unzureichend auch charakterisiert sind. Mit dem erfindungsgemäßen Verfahren kann die Tubulogenese vollständig charakterisiert werden, wobei die Tubuli-Strukturen Polarisationsmarkierungen und Komponenten der extrazellulären Matrix (EZM) enthalten, die bei in vivo proximalen Tubuli vorkommen. Das vorliegende Verfahren ist das erste 3D-Verfahren zur Bildung von den humanen Tubuli ähnlichen Strukturen, die Durchmesser im physiologischen Größenbereich aufweisen.

Ein weiterer Vorteil des Systems ist, dass Hydrogele nach Bedarf durch die Zugabe von Kollagenase abgebaut werden können. Die Tubuli-Zellen können dann weiter durch quantitative Methoden analysiert werden, wie beispielsweise Polymerase-Kettenreaktion (PCR) oder für Durchflusszytometrie, wie FACS (englisch: fluorescence-activated cell sorting). Auf diese Weise können die Tubuli-Zellen auch als Zellquelle für einen Patienten zur Zelltransplantation verwendet werden.

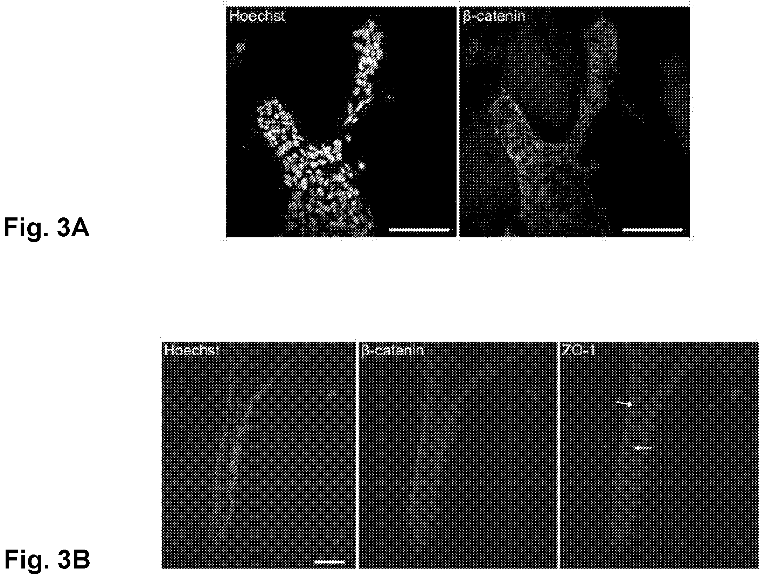

Die **Fig. 3A** zeigt mikroskopische Aufnahmen mit einem Maßstabsbalken von 100 µm, die die Polarisation von HK-2-Zellen nach vier Wochen in der Zellkultur zeigen. Die **Fig. 3B** zeigt mikroskopische Aufnahmen mit einem Maßstabsbalken von 100 µm, die die Immunofluoreszenz von Polarisationsmarkern auf primären humanen proximalen Tubuluszellen nach vier Wochen in der Zellkultur zeigen. Die Pfeile zeigen auf typische enge Verbindungen zwischen den Zellen.

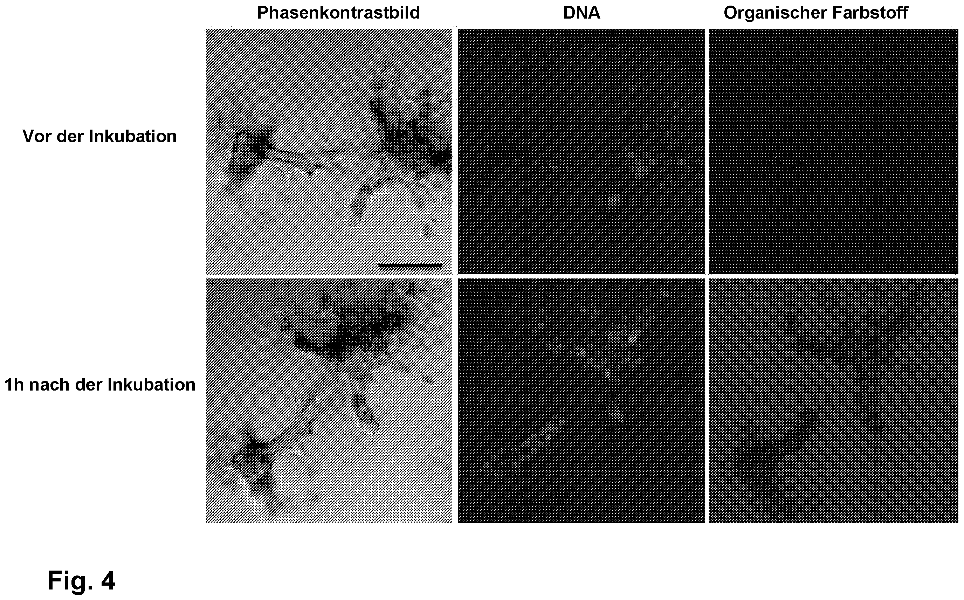

Die **Fig. 4** zeigt eine mikroskopische Aufnahme mit einem Maßstabsbalken von 100 µm und Phasenkontrast und Fluoreszenzbildern der Nierentubuli im Hydrogel vor (oben) und nach der Inkubation mit einem anionischen, organischen Fluoreszenzfarbstoff, dem fluoreszierenden, organischen Anionenfarbstoff Dilithium-4-amino-N-[3-(vinylsulfonyl)phenyl]naphthalimide-3,6-disulfonat mit der Kurzbezeichnung Lucifer Yellow. Die Funktionalität der Tubuli wurde unter anderem durch die Transportfunktion für organische Substanzen nachgewiesen. Zu diesem Zweck wurden die Tubuli entsprechend der Darstellung Fig. 1 (Kultivierung der HK-2-Zellen im Hydrogel) für vier Wochen im Hydrogel aus PEG und Heparin (mit MMP-sensitiven Peptid-Linkern) kultiviert und mit Lichtmikroskopie, das heißt mit Phasenkontrastmikroskopie und konfokaler Laserrastermikroskopie, charakterisiert, wie die Abbildung in **Fig. 4** vor der Inkubation zeigt. Nach Charakterisierung mit Lichtmikroskopie wurde das Zellkulturmedium entfernt und durch Phenolrot-freies Medium mit 100 µM des fluoreszierenden, organischen Anionenfarbstoffs Dilithium-6-Amino-2-(hydrazincarbonyl)-1,3-dioxobenzo[de]isoquinoline-5,8-disulfonat mit der Bezeichnung Lucifer Yellow ersetzt und für eine Stunde inkubiert. Nach der Inkubation wurden das Medium durch farbstofffreies Medium ersetzt und die Tubuli erneut mit konfokaler Laserrastermikroskopie untersucht. Der Fluoreszenzfarbstoff Lucifer Yellow konnte jetzt im Lumen der Tubuli nachgewiesen werden. Dementsprechend sind die organischen Anionentransporter der Tubuli funktionell aktiv. Das heißt, es wurde eine wichtige Funktionalität gesunder Nierentubuli nachgewiesen, wie die **Fig. 4** zeigt.

### Beispiel 1:

Die polymeren Ausgangsstoffe (Prekursoren) für die Hydrogelherstellung bestanden entsprechend wie bei Tsurkan et al., Advanced Materials 2013, vol. 25 (18) pp. 2606-2610**,** beschrieben aus Heparin, funktionalisiert mit sechs Maleimidgruppen (HEP-HM6) mit einem Molekulargewicht von 15.000 g/mol und vierarmigem PEG, funktionalisiert mit enzymatisch spaltbaren Peptidsequenzen an jedem Arm mit einer Gesamtmolmasse von 15.500 g/mol (PEG-MMP). Die PEG-Heparin-Hydrogele wurden hergestellt, wie bei Tsurkan et al., Advanced Materials 2013, vol. 25 (18) pp. 2606-2610**,** beschrieben, mit folgenden Änderungen: 50.000 Zellen (HK-2-Zellen = human kidney proximal tubule epithelial cells (ATCC CRL-2190)) wurden in 12,5 µl HEP-HM6 (0,56 mg HEP-HM6 gelöst in 12,5 µl), welches in phosphatgepufferter Salzlösung (PBS) gelöst ist, gemischt und dann mit 12,5 µl PEG-MMP (0,58 mg PEG-MMP gelöst in 12,5 µl), gelöst in PBS, vermischt um die Gelbildung zu starten. Der Feststoffgehalt betrug 4,4 %, das molare Verhältnis der Gelkomponenten betrug 1:1. Die im Hydrogel eingebetteten Zellen wurden für vier Wochen in DMEM/F-12 Medium (Gibco), welches mit 10% fötalem Rinderserum (FBS, Biochrom) und 1% Penicillin/Streptomycin versetzt wurde, kultiviert. Das Medium wurde sofort nach der Gelbildung und danach aller drei Tage gewechselt. Überaschenderweise formten sich Tubuli ab der dritten Woche, welche mit Lichtmikroskopie charakterisiert wurden, siehe **Fig. 2B** (rechts) und **Fig. 3A****.** Die resultierenden Hydrogele wiesen nach der Gelbildung ein Speichermodul in einem Bereich von 550±250 Pa auf. Das Speichermodul wurde mittels oszilatorischer Rheometrie von in PBS bei Raumtemperatur gequollenen Hydrogelscheiben durch Nutzung eines Rotationsrheometers (ARES LN2; TA Instruments, Eschborn, Germany) mit einer Platte-Platte-Messanordnung mit einem Plattendurchmesser von 25 mm durch frequenzabhängige Messung bei 25 °C in einem Scherfrequenzbereich 10⁻¹ - 10² rad s⁻¹ mit einer Deformationsamplitude von 2% bestimmt.

### Beispiel 2

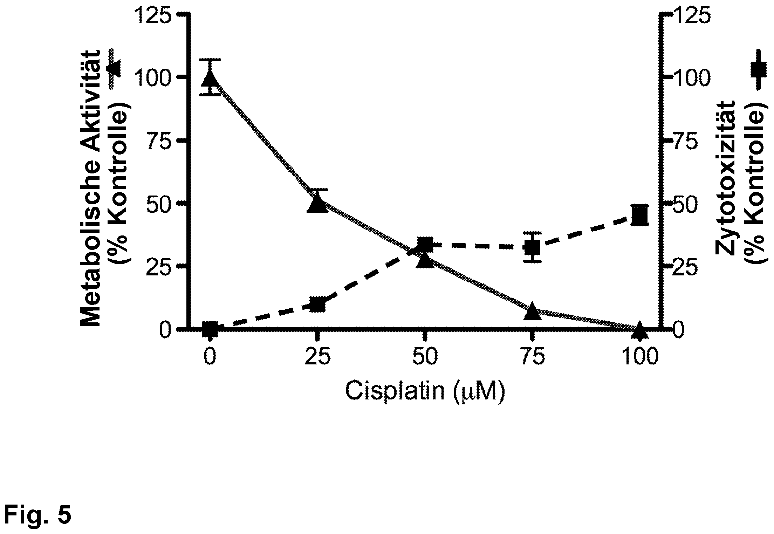

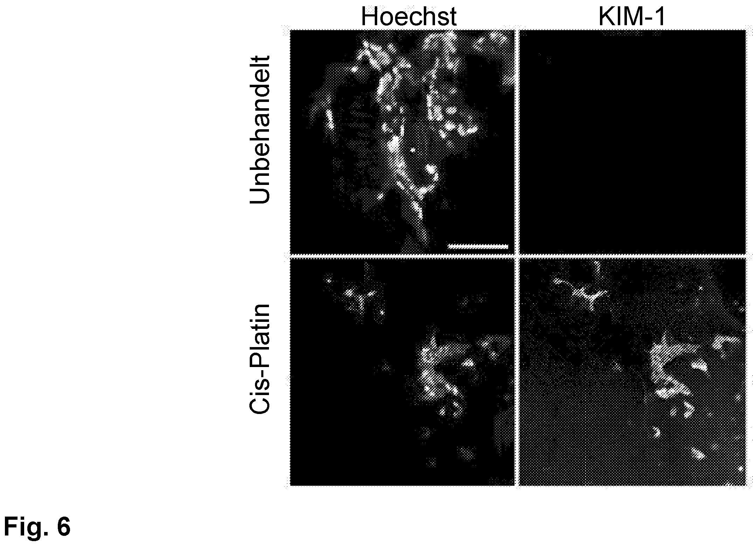

Die vorliegende Erfindung kann als ein Assay für Nephrotoxizität verwendet werden. Dies konnte durch Inkubieren der Tubuli-Strukturen mit dem nephrotoxischen Chemotherapeutikum Cisplatin gezeigt werden. Die Cisplatin-Aufnahme aus den Nierentubuli-Zellen aktiviert Signalwege, die den Zelltod fördern sowie die Produktion von entzündungsfördernden Zytokinen erhöhen, welche ebenfalls zu Verletzungen und zum Zelltod beitragen. Nach vier Wochen in der Kultur, analog Beispiel 1 mit HK-2-Zellen, wurden die Tubuli-Organoide mit serumfreiem Medium, welches 0 bis 100 µM Cisplatin enthält, 48 Stunden lang inkubiert. Nach der Inkubation mit dem Wirkstoff gab es eine dosisabhängige Abnahme der Stoffwechselaktivität, wie in der **Fig. 5** gezeigt. Im Gegensatz dazu stieg die Zytotoxizität der Tubuli mit zunehmender Cisplatin-Dosierung, wie ebenfalls in der **Fig. 5** gezeigt. Cisplatin verursacht auch eine Zunahme der Apoptose, dem Zelltod, der mit Caspase-3-Immunzytochemie beobachtet werden konnte. Wie die **Fig. 6** zeigt, exprimierten die verletzten Strukturen auch den eine Verletzung anzeigenden Nieren-Biomarker KIM 1 welcher auch in vivo genutzt wird um verletzte Nierenstrukturen zu charakterisieren.

### Beispiel 3

Das Verfahren gemäß Beispiel 2 kann auch mit anderen Wirkstoffen, wie Doxorubicin oder Toxinen wie Lipopolysachariden wiederholt werden, um Sepsis zu modellieren. Die Tubuli-Strukturen können mit dem Wirkstoff Doxorubicin, mit einem Gehalt von 0 bis 100 µM, inkubiert werden. Für die Stoffwechsel- und Zytotoxizität- Messungen sind ähnliche Ergebnisse zu erwarten wie in **Fig. 5****.** Der Zelltod und die KIM-1-Expression könnten somit ebenfalls analysiert werden.

### Beispiel 4

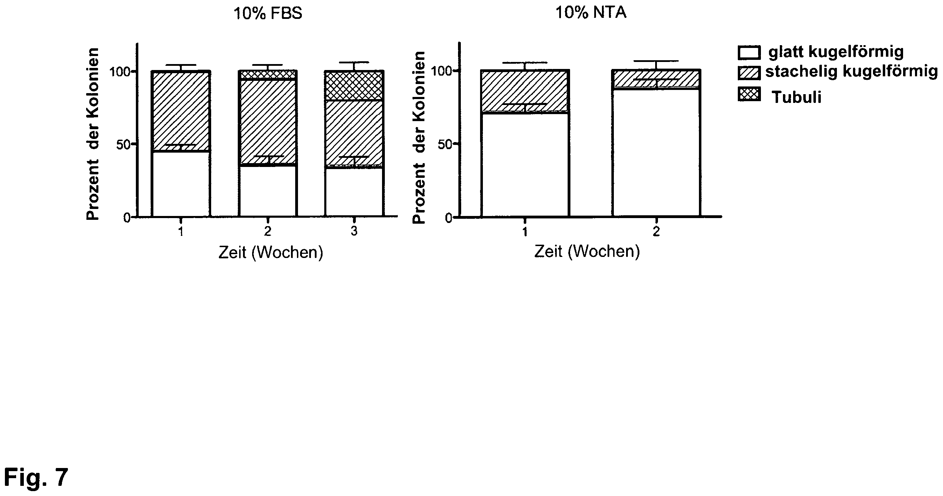

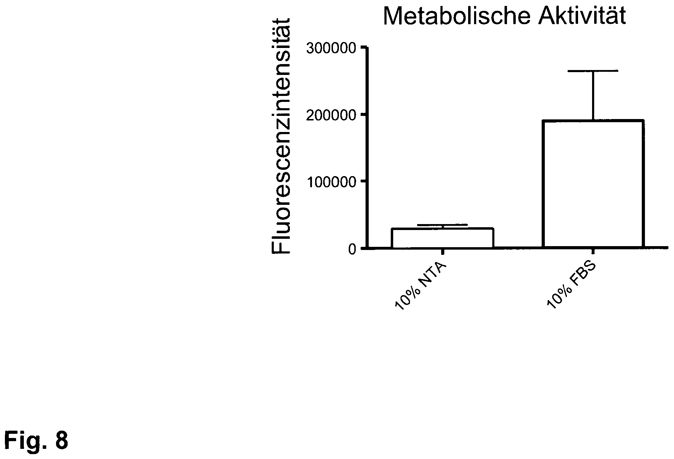

Das Modell kann auch angewandt werden, um zu untersuchen, wie Wachstumsfaktoren die Nieren-Tubulogenese fördern. In diesem Beispiel wurde die Wirkung von Wachstumsfaktoren im Serum sondiert. Panexin NTA der Firma PAN-Biotech, ein Serumersatz, die keine Wachstumsfaktoren enthält, wurde im Medium in der gleichen Konzentration wie das Vergeichsserum (Kontrolle), 10% fötales Rinderserum (FBS), hinzugefügt. Die Tubulogenese wurde während der Dauer der Kultur quantifiziert und die metabolische Aktivität wurde nach drei Wochen in der Kultur gemessen. Wie die **Fig. 7** zeigt, inhibiert der Mangel an Wachstumsfaktoren im Serum Tubulogenese. Die metabolische Aktivität der Zellen wurde ebenfalls durch die Zugabe von 10% FBS erhöht, wie die **Fig. 8** zeigt.

## Patentansprüche

1. Verfahren zur Bildung von Nieren-Tubuli, umfassend die Schritte
> Einbettung von einzelnen Nieren-Zellen in ein synthetisches Hydrogel, basierend auf Polyethylenglykol (PEG) als Komponente, wobei das PEG-Hydrogelsystem über enzymatisch spaltbare Peptide vernetzt wird, und wobei die PEG-Moleküle durch die Peptideinheiten mit Molekülen eines Glykosaminoglykans, ausgewählt aus Heparin, Heparin mit angepasstem Sulfatierungsmuster, Chondroitinsulfat und Hyaluronsäure, unter Hydrogelbildung vernetzt werden, und
> Kultivierung der Zellen bis zur Bildung von Tubulistrukturen.

2. Verfahren nach Anspruch 1, **dadurch gekennzeichnet, dass** die Nieren-Zellen Nierentubuluszelllinien entstammen oder primäre Nierentubuluszellen sind oder aus induziert pluripotenten Stammzellen (iPSCs) oder von mesenchymalen Stammzellen abgeleitete Zellen sind.

3. Verfahren nach Anspruch 2, **dadurch gekennzeichnet, dass** die Nieren-Zellen aus humanen Nierentubuluszelllinien entstammen oder primäre humane Nierentubuluszellen sind oder aus humanen induziert pluripotenten Stammzellen (iPSCs) oder von humanen mesenchymalen Stammzellen abgeleitete Zellen sind.

4. Verfahren nach Anspruch 2 oder 3, **dadurch gekennzeichnet, dass** die Nierenzellen proximale Tubuluszellen oder distale Tubuluszellen oder Sammelrohrzellen sind.

5. Verfahren nach Anspruch 3, **dadurch gekennzeichnet, dass** die Nierentubulus-Zelllinie eine immortalisierte proximale Tubulus-Epithelzelllinie, abgeleitet von normalem adultem humanen Nierengewebe ist.

6. Verfahren nach einem der Ansprüche 3 bis 5, **dadurch gekennzeichnet, dass** die Kultivierung solange fortgesetzt wird, bis die Tubulistrukturen in Größe, Struktur und Morphologie und Funktionalität adulten humanen Nierentubuli entsprechen oder zumindest ähneln.

7. Verfahren nach Anspruch 6, **dadurch gekennzeichnet, dass** die Funktionalität der Nierentubuli durch die Expression humaner renaler Marker, die Ansprechbarkeit auf bekannte nephrotoxische Verbindungen, und daraus folgender Expression renaler Verletzungs- und Apoptose-Marker und dadurch, dass die Tubuli in der Lage sind, anionisch geladene organische Moleküle in das Lumen der Tubuli zu transportieren, beschreibbar ist.

8. Verfahren nach einem der Ansprüche 1 bis 7, **dadurch gekennzeichnet, dass** eine Co-Kultivierung der Tubuluszellen zusammen mit mesenchymalen Stammzellen und/oder Nierenzellen und/oder Endothelzellen erfolgt, die co-lokalisiert mit den Tubuluszellen im Hydrogel vorliegen.

9. Verfahren nach Anpruch 8, **dadurch gekennzeichnet, dass** eine Co-Kultivierung der Tubuluszellen zusammen mit humanen mesenchymalen Stammzellen und/oder humanen Nierenzellen und/oder humanen Endothelzellen erfolgt, die co-lokalisiert mit den Tubuluszellen im Hydrogel vorliegen.

10. Verfahren nach einem der Ansprüche 1 bis 9 **dadurch gekennzeichnet, dass** das Medium 0 % (v/v) Serum enthält.

11. Verfahren nach einem der Ansprüche 1 bis 9, **dadurch gekennzeichnet, dass** das Medium Serum enthält.

12. Anwendung des Verfahrens nach einem der Ansprüche 1 bis 11 zum dreidimensionalen Monitoring der Bildung von humanen Nierentubuli oder Nierentubuli von Säugetieren.

13. Anwendung nach Anspruch 12 zur Charakterisierung des Einflusses der Hydrogelsteifigkeit und/oder des Einflusses des Abbaus des Hydrogels über enzymatisch spaltbare Peptidbrücken und/oder die Charakterisierung des Einflusses der Gegenwart von Glykosaminoglykanen und/oder Peptiden auf die Bildung der Nierentubuli durch Einbau der Glykosaminoglykane und/oder Peptide in das synthetische Hydrogel.

14. Anwendung nach einem der Anspruch 12 oder 13 zur Charakterisierung des Einflusses von löslichen Molekülen oder Wirkstoffen auf die Bildung der Nierentubuli durch Zugabe einer Verbindung zum Kulturmedium oder Einbettung der Komponente in das Hydrogel.

15. Anwendung nach einem der Ansprüche 12 bis 14 für die Analyse der Toxizität einer Verbindung auf Nierentubuli durch Zugabe dieser Verbindung zum Medium oder deren Einbetten in das Hydrogel.

16. Anwendung nach einem der Ansprüche 12 bis 15 zur Untersuchung der Tubulogenese oder der Nephrotoxizität unter Durchführung des Verfahrens in einer 24-, 48- oder 96-Wellplatte.

## Claims

1. A method for forming renal tubules, comprising the steps
> embedding individual renal cells in a synthetic hydrogel, based on polyethylene glycol (PEG) as a component, wherein the PEG hydrogel system is linked via enzymatically cleavable peptides, and wherein the PEG molecules are linked with molecules of a glycosaminoglycan, selected from heparin, heparin with adapted sulfation pattern, chondroitin sulfate and hyaluronic acid, through the peptide units under formation of a hydrogel, and
> cultivating the cells up to the formation of tubule structures.

2. The method according to claim 1, **characterized in that** the renal cells originate from renal tubule cell lines or are primary renal tubule cells or are cells derived from induced pluripotent stem cells (iPSCs) or from mesenchymal stem cells.

3. The method according to claim 2, **characterized in that** the renal cells originate from human renal tubule cell lines or are primary human renal tubule cells or are cells derived from human induced pluripotent stem cells (iPSCs) or from human mesenchymal stem cells.

4. The method according to claim 2 or 3, **characterized in that** the renal cells are proximal tubule cells or distal tubule cells or collecting duct cells.

5. The method according to claim 3, **characterized in that** the renal tubule cell line is an immortalized proximal tubule epithelial cell line derived from normal adult human renal tissue.

6. The method according to one of claims 3 to 5, **characterized in that** the cultivation is continued until the size, structure and morphology and functionality of the tubule structures correspond to adult human renal tubules or are at least similar thereto.

7. The method according to claim 6, **characterized in that** the functionality of the renal tubules can be described by the expression of human renal markers, the responsiveness to familiar nephrotoxic connections and resulting expression of renal injury and apoptosis markers and **in that** the tubules are able to transport anionically charged organic molecules into the lumen of the tubules.

8. The method according to one of claims 1 to 7, **characterized in that** a co-cultivation of the tubule cells is done together with mesenchymal stem cells and/or renal cells and/or endothelial cells which are present in the hydrogel co-localized with the tubule cells.

9. The method according to claim 8, **characterized in that** a co-cultivation of the tubule cells is done together with human mesenchymal stem cells and/or human renal cells and/or human endothelial cells which are present in the hydrogel co-localized with the tubule cells.

10. The method according to one of claims 1 to 9, **characterized in that** the medium contains 0% (v/v) serum.

11. The method according to one of claims 1 to 9, **characterized in that** the medium contains serum.

12. Use of the method according to one of claims 1 to 11 for three-dimensional monitoring of the formation of human renal tubules or renal tubules of mammals.

13. Use according to claim 12 for characterizing the influence of hydrogel stiffness and/or the influence of the degradation of the hydrogel via enzymatically cleavable peptide bridges and/or for characterizing the influence of the presence of glycosaminoglycans and/or peptides on the formation of the renal tubules through integration of the glycosaminoglycans and/or peptides in the synthetic hydrogel.

14. Use according to one of claims 12 or 13 for characterizing the influence of soluble molecules or active ingredients on the formation of the renal tubules through addition of a compound to the culture medium or embedding the component in the hydrogel.

15. Use according to one of claims 12 to 14 for the analysis of the toxicity of a compound to renal tubules through addition of this compound to the medium or its embedding in the hydrogel.

16. Use according to one of claims 12 to 15 for examining the tubulogenesis or the nephrotoxicity by performing the method in a 24, 48 or 96 well plate.

## Revendications

1. Procédé de formation de tubules rénaux, comprenant les étapes suivantes
> incorporation de cellules rénales individuelles dans un hydrogel synthétique, à base de polyéthylèneglycol (PEG) comme composant, le système hydrogel PEG étant réticulé par l'intermédiaire de peptides clivables par voie enzymatique, et les molécules de PEG étant réticulées par les unités peptidiques avec des molécules d'un glycosaminoglycane choisi parmi l'héparine, l'héparine avec un modèle de sulfatation adapté, le sulfate de chondroïtine et l'acide hyaluronique, avec formation d'hydrogel, et
> culture des cellules jusqu'à la formation de structures tubulaires.

2. Procédé selon la revendication 1, **caractérisé en ce que** les cellules rénales proviennent de lignées de cellules tubulaires rénales ou sont des cellules tubulaires rénales primaires ou sont des cellules dérivées de cellules souches pluripotentes induites (iPSC) ou de cellules souches mésenchymateuses.

3. Procédé selon la revendication 2, **caractérisé en ce que** les cellules rénales proviennent de lignées de cellules tubulaires rénales humaines ou sont des cellules tubulaires rénales humaines primaires ou sont des cellules dérivées de cellules souches pluripotentes induites humaines (iPSC) ou de cellules souches mésenchymateuses humaines.

4. Procédé selon la revendication 2 ou 3, **caractérisé en ce que** les cellules rénales sont des cellules tubulaires proximales ou des cellules tubulaires distales ou des cellules tubulaires collectrices.

5. Procédé selon la revendication 3, **caractérisé en ce que** la lignée cellulaire de tubule rénal est une lignée de cellules épithéliales tubulaires proximales immortalisée, dérivée de tissu rénal humain adulte normal.

6. Procédé selon l'une des revendications 3 à 5, **caractérisé en ce que** la culture est poursuivie jusqu'à ce que les structures tubulaires correspondent ou au moins ressemblent à des tubules rénaux humains adultes en termes de taille, de structure et de morphologie et de fonctionnalité.

7. Procédé selon la revendication 6, **caractérisé en ce que** la fonctionnalité des tubules rénaux peut être décrite par l'expression de marqueurs rénaux humains, la sensibilité à des composés néphrotoxiques connus, et l'expression consécutive de marqueurs de lésion rénale et d'apoptose, et **en ce que** les tubules sont capables de transporter des molécules organiques chargées de manière anionique dans la lumière des tubules.

8. Procédé selon l'une quelconque des revendications 1 à 7, **caractérisé en ce qu'**une coculture des cellules tubulaires est réalisée conjointement avec des cellules souches mésenchymateuses et/ou des cellules rénales et/ou des cellules endothéliales, qui sont colocalisées avec les cellules tubulaires dans l'hydrogel.

9. Procédé selon la revendication 8, **caractérisé en ce qu'**une coculture des cellules tubulaires a lieu conjointement avec des cellules souches mésenchymateuses humaines et/ou des cellules rénales humaines et/ou des cellules endothéliales humaines, qui sont colocalisées avec les cellules tubulaires dans l'hydrogel.

10. Procédé selon l'une quelconque des revendications 1 à 9, **caractérisé en ce que** le milieu contient 0 % (v/v) de sérum.

11. Procédé selon l'une quelconque des revendications 1 à 9, **caractérisé en ce que** le milieu contient du sérum.

12. Application du procédé selon l'une quelconque des revendications 1 à 11 pour le suivi tridimensionnel de la formation de tubules rénaux humains ou de tubules rénaux de mammifères.

13. Application selon la revendication 12 pour caractériser l'influence de la rigidité de l'hydrogel et/ou l'influence de la dégradation de l'hydrogel par des ponts peptidiques clivables par voie enzymatique et/ou la caractérisation de l'influence de la présence de glycosaminoglycanes et/ou de peptides sur la formation des tubules rénaux par incorporation des glycosaminoglycanes et/ou des peptides dans l'hydrogel synthétique.

14. Application selon l'une des revendications 12 ou 13 pour caractériser l'influence de molécules solubles ou de principes actifs sur la formation des tubules rénaux par addition d'un composé au milieu de culture ou incorporation du composant dans l'hydrogel.

15. Application selon l'une quelconque des revendications 12 à 14 pour l'analyse de la toxicité d'un composé sur les tubules rénaux par addition dudit composé au milieu ou son incorporation dans l'hydrogel.

16. Application selon l'une quelconque des revendications 12 à 15 pour l'étude de la tubulogenèse ou de la néphrotoxicité, en mettant en oeuvre le procédé dans une plaque à 24, 48 ou 96 puits.
